# NEW EUROPEAN PATENT SPECIFICATION

(11) **EP 1 766 395 B2**
(45) Date of publication and mention of the opposition decision: **19.09.2018**
(45) Mention of the grant of the patent: 17.11.2010
(21) Application number: 05755309.1
(22) Date of filing: 07.06.2005
(51) Int. Cl.: G01N 33/53, G01N 33/536, G01N 33/537, G01N 33/543, G01N 33/551

(54) **METHOD FOR THE EARLY DETECTION OF RENAL DISEASE AND INJURY**
VERFAHREN ZUM FRÜHEN NACHWEIS EINER NIERENKRANKHEIT UND -VERLETZUNG
PROCEDE POUR DETECTER DE MANIERE PRECOCE UNE MALADIE RENALE ET UNE LESION RENALE

(30) Priority: 07.06.2004 US 577662 P; 31.03.2005 US 96113
(43) Date of publication of application: 28.03.2007
(60) Divisional application: 10186256.3
(73) Proprietor: The Children's Hospital Medical Center, Cincinnati, Ohio 45229-3039 (US); The Trustees of Columbia University, New York NY 10027 (US)
(72) Inventor: DEVARAJAN, Prasad, Cincinnati, OH 45242 (US); BARASCH, Jonathan, M., New York, NY 10023 (US)
(74) Representative: Høiberg P/S
(86) International application number: PCT/US2005/019951
(87) International publication number: WO 2005/121788

(56) References cited:
- EP-A- 1 616 184
- EP-A- 1 750 500
- WO-A-2004/005544
- WO-A2-2004/005544
- US-A- 6 136 526
- US-A- 6 136 526
- OHLSSON S ET AL: "Increased circulating levels of proteinase 3 in patients with anti-neutrophilic cytoplasmic autoantibodies-associated systemic vasculitis in remission." CLINICAL AND EXPERIMENTAL IMMUNOLOGY MAR 2003, vol. 131, no. 3, March 2003 (2003-03), pages 528-535, XP002467651 ISSN: 0009-9104
- MATTHAEUS T ET AL: "ACUTE ISCHEMIC RENAL FAILURE INDUCES EXPRESSION OF NEUTROPHIL GELATINASE-ASSOCIATED LIPOCALIN AND MATRIX METALLOPROTEINASE-9 IN DAMAGED TUBULI" KIDNEY AND BLOOD PRESSURE RESEARCH, BASEL, CH, vol. 24, no. 4-6, 29 September 2001 (2001-09-29), page 342, XP009065224 ISSN: 1420-4096
- KJEDSEN L. ET AL: 'Characterization of two ELISAs for NGAL, a newly described lipocalin in human neutrophils' JOURNAL OF IMMUNOLOGICAL METHODS vol. 198, 1996, pages 155 - 164, XP002061581
- MISHRA J. ET AL: 'Neutrophil Gelatinase-Associated Lipocalin: A Novel Early Urinary Biomarker for Cisplatin Nephrotoxicity' AMERICAN JOURNAL OF NEPHROLOGY vol. 24, 12 May 2004, pages 307 - 315, XP008063174
- MISHRA J. ET AL: 'Identification of Neutrophil Gelatinase-Associated Lipocalin as a Novel Early Urinary Biomarker for Ischemic Renal Injury' J. AM. SOC. NEPHROL. vol. 14, 2003, pages 2534 - 2543, XP002995994
- AXELSSON L ET AL: "Studies of the release and turnover of a human neutrophil lipocalin" SCANDINAVIAN JOURNAL OF CLINICAL AND LABORATORY INVESTIGATION, vol. 55, no. 7, 1995, pages 577-588, ISSN: 0036-5513
- KJELDSEN LARS ET AL: "Isolation and characterization of gelatinase granules from human neutrophils" BLOOD, vol. 83, no. 6, 1994, pages 1640-1649, ISSN: 0006-4971
- KJELDSEN LARS ET AL: "Identification of neutrophil gelatinase-associated lipocalin as a novel matrix protein of specific granules in human neutrophils" BLOOD, vol. 83, no. 3, 1994, pages 799-807, ISSN: 0006-4971
- DATABASE BIOSIS [Online] BIOSCIENCES INFORMATION SERVICE, PHILADELPHIA, PA, US; 1995, BLAESER JOERG ET AL: "A sandwich enzyme immunoassay for the determination of neutrophil lipocalin in body fluids" Database accession no. PREV199598308194 & CLINICA CHIMICA ACTA, vol. 235, no. 2, 1995, pages 137-145, ISSN: 0009-8981
- BLÄSER J. ET AL: 'A sandwich enzyme immunoassay for the determination of neutrophil lipocalin in bod fluids' CLINICA CHIMICA ACATA vol. 235, 1995, pages 137 - 145
- XU S.Y. ET AL: 'Serum measurements of human neutrophil lipocalin (HNL) discriminate between acute bacterial and viral infections' SCAND. J. CLIN. LAB. INVEST. vol. 55, 1995, pages 125 - 131
- KJELDSEN L. ET AL: 'Chatachterization of two ELISAs for NGAL, a newly described lipocalin in human neutrophils' JOURNAL OF IMMUNOLOGICAL METHODS vol. 198, 1996, pages 155 - 164
- MISHRA J. ET AL: 'Neutrophil gelatinase-associated lipocalin: A novel early urinary biomarker for cisplatin nephrotoxicity' AM. J. NEPHROL vol. 24, 2004, pages 307 - 315
- OHLSSON S. ET AL: 'Increased circulating levels of proteinase 3 in patients with anti-neutrophilic cytoplasmic autoantibodies-associated systemic vasculitis in remission' CLIN. EXP. IMMUNOL. vol. 131, 2003, pages 528 - 535

## Description

### BACKGROUND OF THE INVENTION

Acute renal failure (ARF) secondary to a renal tubular cell injury, including an ischemic injury or a nephrotoxic injury remains a common and potentially devastating problem in clinical medicine and nephrology, with a persistently high rate of mortality and morbidity despite significant advances in supportive care. Pioneering studies over several decades have illuminated the roles of persistent vasoconstriction, tubular obstruction, cellular structural and metabolic alterations, and the inflammatory response in the pathogenesis of ARF. While these studies have suggested possible therapeutic approaches in animal models, translational research efforts in humans have yielded disappointing results. The reasons for this may include the multifaceted response of the kidney to ischemic injury and nephrotoxins, and a paucity of early biomarkers for ARF with a resultant delay in initiating therapy.

An individual is considered to have acute renal failure when the patient's .. serum creatinine value either (1) increased by at least 0.5 mg/dL when the baseline serum creatinine level was less than 2.0 mg/dL; (2) increased by at least 1.5 mg/dL when the baseline serum creatinine level was greater than or equal to 2.0 mg/dL; or (3) increased by at least 0.5 mg/dL, regardless of the baseline serum creatinine level, as a consequence of exposure to radiographic agents.

It is believed that introduction of therapy early in the disease process will reduce the mortality rate associated with ARF and shorten the time for treatment of various types of renal tubular cell injuries, including, but not limited to, ischemic and nephrotoxic renal injuries. The identification of a reliable, early biomarker for a renal tubular cell injury would be useful to facilitate early therapeutic intervention, and help guide pharmaceutical development by providing an indicator of nephrotoxicity.

The traditional laboratory approach for detection of renal disease involved determining the serum creatinine, blood urea nitrogen, creatinine clearance, urinary electrolytes, microscopic examination of the urine sediment, and radiological studies. These indicators are not only insensitive and nonspecific, but also do not allow for early detection of the disease. Indeed, while a rise in serum creatinine is widely considered as the "gold standard" for the detection of ARF, it is now clear that as much as 50% of the kidney function may already be lost by the time the serum creatinine changes.

A few urinary biomarkers for ischemic renal injury have been earlier described, including kidney injury molecule-1 (KIM-1) and cysteine rich protein 61 (Cyr61)). KIM-1 is a putative adhesion molecule involved in renal regeneration. In a rat model of ischemia-reperfusion injury, KIM-1 was found to be upregulated 24-48 hours after the initial insult, rendering it a reliable but somewhat late marker of tubular cell damage. Recent studies have shown that KIM-1 can be detected in the kidney biopsy and urine of patients with ischemic acute tubular necrosis. However, this detection was documented in patients with established ischemic renal damage, late in the course of the illness. The utility of urinary KIM-1 measurement for the detection of early ARF or subclinical renal injury has thus far not been validated.

The protein Cyr61 was found to be a secreted cysteine-rich protein that is detectable in the urine 3-6 hours after ischemic renal injury in animal models. However, this detection required a bioaffinity purification and concentration step with heparin-sepharose beads, followed by a Western blotting protocol. Even after bioaffinity purification several nonspecific cross-reacting peptides were apparent. Thus, the detection of Cyr61 in the urine is problematic with respect to specificity as well as the cumbersome nature of the procedure.

Therefore, there remains an urgent need to identify improved biomarkers for early ischemic and nephrotoxic renal injuries.

Ohlson S. et al, Clinical And Experimental Immunology Mar 2003, vol. 131, no. 3, March 2003, pages 528-535 describe how circulating levels of proteinase 3 are increased in patients with anti-neutrophilic cytoplasmic autoantibodies-associated systemic vasculitis in remission. Other markers were also measured.

Matthaeus T. et al, Kidney And Blood Pressure Research, vol 24, no. 4-6, 29 September 2001 page 342 describe how acute ischemic renal failure induces expression of Neutrophil Gelatinase-Associated Lipocalin and Matrix Metalloproteinase-9 in Damaged Tubuli.

EP-A-1 750 500 relates to neutrophil gelatinase-associated lipocalin (NGAL) and its use in compositions and methods for treating, reducing, ameliorating, or preventing a condition, injury or disease, typically selected from an ischemic, an ischemia-reperfusion, or a toxin-induced injury in an organ.

EP-A-1 616 184 describes a method for detecting renal tubular cell injury in a mammal by obtaining a urine sample and contacting it with an antibody for the renal tubular cell injury marker NGAL to allow formation of a complex of the antibody and marker, and detecting the complex.

Kjeldsen L. et al, Journal Of Immunological Methods vol 198, 1996, pages 155-164 describe NGAL, a newly described member of the lipocalin protein family, secreted from specific granules of human neutrophils upon activation of the cells.

Mishra J. et al, American Journal of Nephrology, vol 24 12 May 2004, pages 307-315 describe how cisplatin is a widely used chemotherapeutic agents which has the risk of nephrotoxicity. This study indicates that NGAL could be an early and quantitative urinary biomarker for cisplatin nephrotoxicity.

Mishra J. et al, J. Am. Soc. Nephrol. Vol 14, 2003, pages 2534-2543 describe identification of neutrophil gelatinase-associated lipocalin as a novel early urinary biomarker for ischemic renal injury.

US 6,136,526 relates to the use of human neutrophil lipocalin (HNL) as a diagnostic marker, for diagnosis in connection with inflammation that may have a bacterial origin. It is put forward that the determination of the HNL level in a sample from a patient assists in discriminating between bacterial and viral infection.

### SUMMARY OF THE INVENTION

The present invention relates to a method for the immediate or early on-set detection of a renal tubular cell injury in a mammalian subject, comprising the steps of: 1) obtaining a blood serum sample from a mammalian subject; 2) determining from the serum sample the level of a biomarker selected from an immediate renal tubular cell injury biomarker, an early on-set renal tubular cell injury biomarker, and mixtures thereof, and (c) evaluating the renal tubular cell injury status of the subject.

The present invention also relates to a method for the immediate or early-onset detection of a renal tubular cell injury in a mammal, comprising the steps of: 1) obtaining a blood serum sample from a mammalian subject; 2) contacting the serum sample with an antibody for an renal tubular cell injury biomarker, the renal tubular cell injury biomarker comprising NGAL, to allow formation of a complex of the antibody and the renal tubular cell injury biomarker; and 3) detecting the antibody-biomarker complex.

The present invention relates to a method for monitoring the effectiveness of a treatment for renal tubular cell injury, comprising the steps of: 1) obtaining a blood serum sample from a mammalian subject experiencing renal tubular cell injury; 2) determining from the serum sample the level of a biomarker selected from an immediate renal tubular cell injury biomarker, an early on-set renal tubular cell injury biomarker, and mixtures thereof, and (c) evaluating the renal tubular cell injury status of the subject.

The invention relates to a method of monitoring the effectiveness of a treatment for renal tubular cell injury comprising the steps of: 1) providing a treatment to a mammalian subject experiencing renal tubular cell injury; 2) obtaining at least one post-treatment serum sample from the subject; and 3) detecting for the presence in the post-treatment serum sample of a biomarker for renal tubular cell injury selected from an immediate renal tubular cell injury biomarker, an early on-set renal tubular cell injury biomarker, and mixtures thereof.

The present invention relates to the use of a kit for detecting the presence of an immediate or early onset biomarker for renal tubular cell injury, comprising: 1) a means for acquiring a quantity of a blood serum sample; and 2) an assay for the detection of early onset biomarker.

The invention further relates to the use of a kit for detecting the presence of an immediate or early onset biomarker for renal tubular cell injury in the serum of a subject, comprising: 1) a means for acquiring a quantity of a blood serum sample; 2) a media having affixed thereto a capture antibody capable of complexing with a renal tubular cell injury biomarker selected from an immediate renal tubular cell injury biomarker, an early on-set renal tubular cell injury biomarker, and mixtures thereof; and 3) an assay for the detection of a complex of the renal tubular cell injury biomarker and the capture antibody.

The invention also relates to the use of a competitive enzyme linked immunosorbent assay (ELISA) kit for determining the renal tubular cell injury status of a mammalian subject, comprising a first antibody specific to a biomarker selected from an immediate renal tubular cell injury biomarker, an early on-set renal tubular cell injury biomarker, and mixtures thereof, to detect its presence in a serum sample of the subject.

The invention further relates to a method of identifying the extent of a renal tubular cell injury caused by an event, comprising: 1) obtaining at least one serum sample from a mammalian subject; 2) detecting in the serum sample the presence of an early-onset biomarker for renal tubular cell injury; and 3) determining the extent of renal tubular cell injury based on the time for on-set of the presence in the serum sample of a biomarker selected from an immediate renal tubular cell injury biomarker, an early on-set renal tubular cell injury biomarker, and mixtures thereof, relative to the time of the event.

The present invention relates to a method for the detection of a renal tubular cell injury in a mammalian subject, comprising the steps of: 1) obtaining a blood serum sample from a mammalian subject comprising up to 1 milliliter from a mammalian subject: following a suspected renal tubular cell injury; 2) determining from the serum sample the level of a biomarker selected from an immediate renal tubular cell injury biomarker, an early on-set renal tubular cell injury biomarker, and mixtures thereof, and (c) evaluating the renal tubular cell injury status of the subject.

The present invention further relates to a method for the detection of a renal tubular cell injury in a mammalian subject, comprising the steps of: 1) obtaining a blood serum sample comprising up to 1 milliliter from a mammalian subject following a suspected a biomarker for a biomarker selected from an immediate renal tubular cell injury biomarker, an early on-set renal tubular cell injury biomarker, and mixtures thereof, to allow formation of a complex of the antibody and the biomarker; and 3) detecting the antibody-biomarker complex.

In this invention the early on-set renal tubular cell injury biomarker is NGAL and the immediate tubular cell renal injury biomarker is NGAL.

This invention is
1. A method for the immediate or early-onset detection of a renal tubular cell injury (RTCI) in a mammal, the RTCI is acute and can be triggered by an ischemic renal injury or ACUTE nephrotoxic injury, by determining the level of the immediate and early onset RTCI biomarker neutrophil gelatinase-associated lipocalin (NGAL) which appears within the first 24 hours of onset of RTCI in blood serum, comprising:
   1) contacting a sample of blood serum obtained from a mammalian subject with an antibody for NGAL, to allow formation of a complex of the antibody and NGAL; and
   2) detecting the antibody-NGAL complex.
2. The method of 1 further comprising:
   3) determining that the mammal has the RTCI, based on the detected complex.
3. The method according to 1, wherein the NGAL appears in blood serum within the first 6 hours of, optionally within 2 hours of and preferably almost immediately after, the onset of RTCI.
4. The method according to 1 to 3, wherein the sample is obtained within 24 hours of the onset of the RTCI or within 24 hours after the suspected RTCI.
5. The method according to any one of 1 to 4, wherein the method is carried out on a plurality of samples from the subject obtained intermittently.
6. The method according to any one of 1 to 5, wherein the step of detecting the antibody-NGAL complex comprises contacting the complex with a second antibody for detecting the NGAL biomarker.
7. The method according to any one of 1 to 6, further comprising the step of detecting the level of NGAL in at least one sample obtained from the subject after the subject has received a treatment for the renal tubular cell injury.
8. The method according to any one of 1 to 7, wherein the step of detecting the antibody-NGAL complex comprises the steps of:
   1) separating any unbound material of the sample from the capture antibody-NGAL complex;
   2) contacting the capture antibody-NGAL complex with a second antibody for detecting NGAL, to allow formation of a complex between NGAL and the second antibody;
   3) separating any unbound second antibody from the NGAL-second antibody complex; and
   4) detecting the second antibody of the NGAL-second antibody complex.
9. The method of any one of 1 to 6, for identifying the extent of the renal tubular cell injury that has been caused by an event, wherein the extent of the renal tubular cell injury is identified based on the time for onset of the presence of NGAL in the sample, relative to the time of the event,
   wherein the event preferably is selected from a surgical procedure, diminished blood supply to the kidneys, impaired heart function, surgical procedures, patients in intensive care units, and the administration of pharmaceuticals, radiocontrast dyes, or other medicament substances to the subject.
10. The method according to any one of 1 to 9, wherein the sample has a volume of up to 1 milliliter.
11. The method according to any one of 1 to 10, wherein the renal tubular cell injury is an acute ischemic injury.
12. The method according to any of 1 to 10, wherein the renal tubular cell injury is an acute nephrotoxic renal injury (NRI).
13. The method according to any one of 1 to 12, wherein the NGAL level is used to evaluate the renal tubular cell injury status of the subject.
14. The method according to any one of 1 to 10 for the rapid diagnosis of acute renal failure (ARF) in patients who are at risk of developing ARF in cardiac surgery.
15. The method according to any of 1 to 10, wherein the appearance of NGAL in serum precedes the increase in serum creatinine.
16. The method according to any of 1 to 10, detecting the onset of RTCI caused by an event selected from the group consisting of all varieties of diminished blood supply to the kidneys, impaired heart function, surgical procedures, patients in intensive care units, and the administration of pharmaceuticals, radiocontrast dyes, or other medicament substances to a subject or nephrotoxins including cancer chemotherapy agents, antibiotics, antifungal agents, anti-inflammatory agents, immunosuppressants, and radiocontrast agents.
17. The method according to any one of 1 to 10, wherein the subject is a patient who is at risk of developing ARF.
18. The method according to any one of 1 to 10 for alerting a clinician to institute maneuvers aimed at preventing progression of a subtle, subclinical renal tubular cell injury to overt ARF.
19. The method according to any one of 1 to 10 for the early detection of the onset of renal tubular cell injury to reduce the time for treatment of the injury and reduce the risk of developing clinical acute renal failure (ARF).
20. The method according to any one of 1 to 10 for assessing the extent of renal injury wherein the extent of the renal injury is based on a proportional relationship between the extent of the injury with the quantity of NGAL present in the blood serum of the subject.
21. The method according to 20, wherein the extent of the renal injury ranges from the very onset of the RTCI to clinical acute renal failure (ARF).

### BRIEF DESCRIPTION OF THE DRAWINGS

FIGURE 1 shows Western analysis of urine NGAL in (Left Panel) samples obtained at various times as shown after CPB from a subject who subsequently developed, ARF, and (Right Panel) recombinant human NGAL standards. Molecular weights in kDa are along the left margin.
FIGURE 2 shows urine NGAL (in ng/ml) at various times after CPB in patients who subsequently developed ARF (upper line, ARF) versus those who did not (lower line, No ARF). The bar represents the time when the initial rise in serum creatinine was detected.
FIGURE 3 shows urine NGAL values of FIGURE 2 corrected for urine creatinine excretion.
FIGURE 4 shows urine NGAL (in ng/ml) at various times after CPB in patients who subsequently developed ARF (upper line, ARF) versus those who did not (lower line, No ARF), determined by ELISA. The bar represents the time when the initial rise in serum creatinine was detected.
FIGURE 5 shows urine NGAL values of FIGUIRE 4 corrected for urine creatinine excretion.
FIGURE 6 shows a scatter graph of all urine NGAL measurements at 2 hours post CPB. An arbitrary dashed line at 50 ng/ml illustrates the separation of values in patients who developed ARF versus those with No ARF.
FIGURE 7 shows serum NGAL (ng/ml) at various times after CPB in patients who subsequently developed ARF (upper line, ARF) versus those who did not (lower line, No ARF), determined by ELISA. The bar represents the time when the initial rise in serum creatinine was detected.
FIGURE 8 shows a scatter graph of all serum NGAL measurements at 2 hours post CPB in patients who developed ARF versus those with No ARF.
FIGURE 9 shows receiver operating characteristic (ROC) curves to determine the discriminatory power of NGAL measurements for the early diagnosis of acute renal injury, with an ROC curve for urine NGAL at 2 hours post CPB.
FIGURE 10 shows receiver operating characteristic (ROC) curves to determine the discriminatory power of NGAL measurements for the early diagnosis of acute renal injury, with an ROC curve for serum NGAL at 2 hours post CPB.

### DETAILED DESCRIPTION OF THE INVENTION

As used herein the expression "renal tubular cell injury" shall mean a renal or kidney failure or dysfunction, either sudden (acute) or slowly declining over time (chronic), that can be triggered by a number of disease or disorder processes, including (but not limited to) renal tubular cell injury; ischemic renal injury (IRI), including acute ischemic injury and chronic ischemic injury; acute renal failure; acute nephrotoxic renal injury (NRI); toxicity, including sepsis (infection), shock, trauma, kidney stones, kidney infection, drug toxicity, poisons or toxins, or after injection with an iodinated contrast dye (adverse effect); and for chronic nephrotoxic renal injury: long-standing hypertension, diabetes, congestive heart failure, lupus, or sickle cell anemia. Both forms of renal failure can result in a life-threatening metabolic derangement.

As used herein the expression "immediate" in relation to a renal tubular cell biomarker is a biomarker protein that can appear in the blood serum within 2 hours of the onset of renal tubular cell injury.

As used herein the expression "early on-set" in relation to a renal tubular cell biomarker is a biomarker protein that can appear in the blood serum within the first 24 hours, more typically within the first 6 hours, of the onset of renal tubular cell injury.

The present invention provides a method for assaying the presence of a renal tubular cell injury biomarker present in the blood serum of a subject at the early onset of renal tubular cell injury. Early detection of the onset of the injury can reduce the time for treatment of the injury, and can reduce the risk of developing clinical acute renal failure (ARF).

A simple point-of-care kit that uses principles similar to the widely-used blood glucose testing kits, for the rapid detection of serum NGAL at the bedside will allow the clinician to rapidly diagnose ARF, and to rapidly institute proven and effective therapeutic and preventive measures. The use of the kit can represent the standard of care for all patients who are at risk of developing ARF, including use in cardiac surgery, kidney transplantation, stroke, trauma, sepsis, dehydration, and nephrotoxins (antibiotics, anti-inflammatory agents, radio-contrast agents, and chemotherapeutic agents). In current clinical practice, when ARF occurs in the setting of these predisposing conditions, the diagnosis is very delayed, and the associated mortality and morbidity unacceptably high. Ironically, even tragically, effective preventive and therapeutic measures are widely available, but almost never administered in a timely manner due to the lack of early biomarkers of ARF. It is anticipated that multiple serial measurements of NGAL will be become indispensable not only for diagnosing and quantifying the initial kidney injury, but also for following the response to early treatment, and for predicting long term outcome.

The biomarker for renal tubular cell injury (which will also be referred to as RTCI biomarker) can be an immediate RTCI biomarker, such as NGAL, which can appear in the blood serum within 2 hours of the onset of renal tubular cell injury. An immediate RTCI biomarker can, as in the case of NGAL, be present in the blood serum of a subject almost immediately after the onset of renal tubular cell injury. The RTCI biomarker can also be an early-onset RTCI biomarker that can appear within the first 24 hours, more typically within the first 6 hours, of the onset of renal tubular cell injury. As such, NGAL is also an example of an early-onset RTCI biomarker.

An effective RTCI biomarker is typically a secreted protein, whereby it can be excreted by the kidney into the urine or transported within the blood serum. An effective RTCI biomarker is also typically a protease-resistant protein, such as NGAL. Nevertheless, an RTCI biomarker can also be a protease-sensitive protein, so long as stable fragments of the protein can be detected in the urine or in the blood serum, such as by antibodies as described hereinafter for NGAL.

The RTCI biomarker can be an ischemic renal injury biomarker (IRI biomarker), a nephrotoxic renal injury biomarker (NRI biomarker), or a mixture thereof. NGAL is an example of both an IRI biomarker and an NRI biomarker.

The method of the invention can be used to detect the onset of renal tubular cell injury, and to monitor the treatment thereof, for a wide variety of events that can include all varieties of diminished blood supply to the kidneys, impaired heart function, surgical procedures, patients in intensive care units, and the administration of pharmaceuticals, radiocontrast dyes, or other medicament substances to a subject: The renal tubular cell injury can be an ischemic renal injury, a nephrotoxic renal injury, or other injury that affects the tubular cells of the kidney. The event can include administration or ingestion of a large and wide variety of nephrotoxins, including, but not limited to cancer chemotherapy (cisplatin, cyclophosphamide, isosfamide, methotrexate), antibiotics (gentamicin, vancomycin, tobramycin), antifungal agents (amphotericin), anti-inflammatory agents (NSAIDs), immunosuppressants (cyclosporine, tacrolimus), and radiocontrast agents. The method can be used to evaluate the nephrotoxicity of both newly-developed and well-known compounds.

The invention also provides a method and describes the use of a kit for assessing the extent of renal injury based on a proportional relationship between the extent of injury, which can range from the very onset of renal tubular cell injury, to clinical ARF, with the quantity of NGAL present in the blood serum of the subject. The invention provides a means for a clinician to estimate the degree of renal injury at an initial assessment, and to monitor the change in status of the injury (worsening, improving, or remaining the same) based on the detected amount of NGAL in the blood serum.

Typically, the clinician would establish a protocol of collecting and analyzing a quantity of fresh blood samples sample from the patient at selected intervals. Typically the blood sample is obtained intermittently during a prescribed period. The period of time between intermittent sampling may be dictated by the condition of the subject, and can range from a sample each 24 hours to a sample taken continuously, more typically from each 4 hours to each 30 minutes. A serum sample is then typically isolated from the blood sample by well known means.

Using the methods and techniques described herein, both a qualitative level of the RTCI biomarker present in the serum can be analyzed and estimated, and a quantitative level of RTCI biomarker present in the serum can be analyzed and measured. The clinician would select the qualitative method, the quantitative method, or both, depending upon the status of the patient. Typically, the quantity of blood serum to be collected is less than 1 milliliter, and more typically less than 10 µl. A typical sample can range from about 1 µl to about 1 ml. Typically the larger quantities of a blood serum sample (about 1 ml) are used for quantitative assays. Typically, these small amounts of serum are easily and readily available from clinical subjects who are either prone to developing ARF, or have developed ARF.

Once an indication of renal tubular cell injury or acute renal failure has been detected, and intervention and treatment of the disease or condition has commenced, the clinician can employ the method and kit of the invention to monitor the progress of the treatment or intervention. If a treatment or surgery that might cause renal tubular cell injury is planned, the clinician can obtain a pretreatment serum sample to determine a baseline value for an individual. Typically, one or more subsequent post-treatment serum samples will be taken and analyzed for the presence of the RTCI biomarker as the treatment of the renal injury commences and continues. If a baseline value was obtained, these post-treatment values can be compared to the baseline value to determine the relative condition of the patient. The treatment is continued until the presence of the RTCI biomarker in subsequent post-treatment serum samples is not detected. As the treatment and intervention ameliorate the condition, the expression of RTCI biomarker, and its presence in the serum, will be correspondingly reduced. The degree of amelioration will be expressed by a correspondingly reduced level of RTCI biomarker, such as NGAL, detected in a sample. As the renal injury nears complete healing, the method can be used to detect the complete absence of the RTCI biomarker, signaling the completion of the course of treatment. Studies with animal models of ischemic or nephrotoxic injury event demonstrated that NGAL is produced in renal tubular cells within minutes following the event. As shown in the examples of the present invention, the NGAL expressed by renal tubular cells rapidly accumulates in the blood, and can be detected far earlier than current diagnostic tests available to indicate renal cell damage. Since NGAL is easily detected in the serum within 2 hours of the renal injury or nephrotoxic treatment, the invention is suitable for use as an early-onset diagnostic. NGAL testing of serum samples from a subject can begin within 30 minutes of a suspected injury, since NGAL begins to appear in the serum at low levels, and continues to rise thereafter. Therefore, it is also of great value to initiate testing at any time within 2 hours of a suspected injury, when NGAL is clearly apparent in serum. Furthermore, it is of value to test at any other time during the first 24 hours following a suspected injury, since NGAL is a highly reliable and easily measured marker of injury that appears in the serum before changes in other parameters, such as creatinine, can be detected. The most highly preferred course of NGAL testing is to collect samples at intervals throughout the course of treatment to monitor real time changes in renal health status.

Both monoclonal and polyclonal antibodies that bind an RTCI biomarker are useful in the methods of the present invention. The antibodies can be prepared by methods known in the art. Monoclonal antibodies for a preferred RTCI biomarker, NGAL, are described, for example, in "Characterization of two ELISAs for NGAL, a newly described lipocalin in human neutrophils", Lars Kjeldsen et al., (1996) Journal of Immunological Methods,. Vol. 198, 155-16. Examples of monoclonal antibodies for NGAL can be obtained from the Antibody Shop, Copenhagen, Denmark, as HYB-211-01, HYB-211-02, and NYB-211-05. Typically, HYB-211-01 and HYB-211-02 can be used with NGAL in both its reduced and unreduced forms. An example of a polyclonal antibody for NGAL is described in "An Iron Delivery Pathway Mediated by a Lipocalin", Jun Yang et al., Molecular Cell, (2002), Vol. 10, 1045-1056. To prepare this polyclonal antibody, rabbits were immunized with recombinant gel-filtered NGAL protein. Sera were incubated with GST-Sepharose 4B beads to remove contaminants, yielding the polyclonal antibodies in serum, as described by the applicants in Jun Yang et al., Molecular Cell (2002).

Typically, the step of detecting the complex of the capture antibody and the RTCI biomarker comprises contacting the complex with a second antibody for detecting the biomarker.

The method for detecting the complex of the RTCI biomarker and the primary antibody comprises the steps of separating any unbound material of the serum sample from the capture antibody-biomarker complex; contacting the capture antibody-biomarker complex with a second antibody for detecting the RTCI biomarker, to allow formation of a complex between the RTCI biomarker and the second antibody; separating any unbound second antibody from the RTCI biomarker-second antibody complex; and detecting the second antibody of the RTCI biomarker-second antibody complex.

A kit for use in the method typically comprises a media having affixed thereto the capture antibody, whereby the serum sample is contacted with the media to expose the capture antibody to NGAL contained in the sample. The kit includes an acquiring means that can comprise an implement, such as a spatula or a simple stick, having a surface comprising the media. The acquiring means can also comprise a container for accepting the serum sample, where the container has a serum-contacting surface that comprises the media. In an another typical embodiment, the assay for detecting the complex of the RTCI biomarker and the antibody can comprise an ELISA, and can be used to quantitate the amount of NGAL in a serum sample. In an alternative embodiment, the acquiring means can comprise an implement comprising a cassette containing the media.

Early detection of the RTCI biomarker can provide an indication of the presence of the protein in a serum sample in a short period of time. Generally, a method and a kit of the present invention can detect the RTCI biomarker in a sample of serum within four hours, more typically within two hours, and most typically within one hour, following renal tubular cell injury. Preferably, the RTCI biomarker can be detected within about 30 minutes following renal tubular cell injury.

A method of the present invention for detecting the RTCI biomarker can be made by adapting the methods and kits known in the art for the rapid detection of other proteins and ligands in a biological sample. Examples of methods and kits that can be adapted to the present invention are described in US Patent 5,656,503, issued to May et al. on August 12, 1997, US Patent 6,500,627, issued to O'Conner et al. on December 31, 2002, US Patent 4,870,007, issued to Smith-Lewis on September 26, 1989, US Patent 5,273,743, issued to Ahlem et al. on December 28, 1993, and US Patent 4,632,901, issued to Valkers et al. on December 30, 1986, all such references being hereby incorporated by reference.

A rapid one-step method of detecting the RTCI biomarker can reduce the time for detecting the renal tubular cell injury. A typical method can comprise the steps of: obtaining a blood serum sample suspected of containing the RTCI biomarker; mixing a portion of the sample with a detecting antibody which specifically binds to the RTCI biomarker, so as to initiate the binding the detecting antibody to the RTCI biomarker in the sample; contacting the mixture of sample and detecting antibody with an immobilized capture antibody which specifically binds to the RTCI biomarker, which capture antibody does not cross-react with the detecting antibody, so as to bind the detecting antibody to the RTCI biomarker, and the RTCI biomarker to the capture antibody, to form a detectable complex; removing unbound detecting antibody and any unbound sample from the complex; and detecting the detecting antibody of the complex. The detectable antibody can be labeled with a detectable marker, such as a radioactive label, enzyme, biological dye, magnetic bead, or biotin, as is well known in the art. The detectable antibody can be attached to a supporting material, such as a membrane, plastic strip, plastic laboratory plate such as those used for ELISA or other high-throughput assays, or any other supporting material, such as those used in other diagnostic kits well known in the art.

To identify potential genes and their proteins that may accompany and mark the earliest onset of renal tubular cell injuries, such as ischemic and nephrotoxic renal injuries, a cDNA microarray assay can be used to detect which of a large number of potential gene targets are markedly upregulated. Utilizing this screening technique, neutrophil gelatinase-associated lipocalin (NGAL) was identified as a gene whose expression is upregulated more than 10 fold within the first few hours following an ischemic renal injury in a mouse model.

NGAL belongs to the lipocalin superfamily of over 20 structurally related secreted proteins that are thought to transport a variety of ligands within a β-barreled-calyx. Human NGAL was originally identified as a 25 kDa protein covalently bound to gelatinase from human neutrophils, where it represents one of the neutrophil secondary granule proteins. Molecular cloning studies have revealed human NGAL to be similar to the mouse 24p3 gene first identified in primary cultures of mouse kidneys that were induced to proliferate. NGAL is expressed at very low levels in other human tissues, including kidney, trachea, lungs, stomach, and colon. NGAL expression is markedly induced in stimulated epithelia. For example, it is upregulated in colonic epithelial cells in areas of inflammation or neoplasia, but is absent from intervening uninvolved areas or within metastatic lesions. NGAL concentrations are elevated in the serum of patients with acute bacterial infections, the sputum of subjects with asthma or chronic obstructive pulmonary disease, and the bronchial fluid from the emphysematous lung. In all these cases, NGAL induction is postulated to be the result of interactions between inflammatory cells and the epithelial lining, with upregulation of NGAL expression being evident in both neutrophils and the epithelium.

It is believed that the detected NGAL induction represents a novel intrinsic response of the kidney proximal tubule cells to renal tubular cell injuries, including both ischemic and nephrotoxic injuries, and is not derived merely from activated neutrophils. First, the response is rapid, with NGAL appearing in the serum within 2 hours of the injury following renal artery occlusion, while renal neutrophil accumulation in this model of ischemic ARF is usually first noted at 4 hours after injury. Second, the temporal patterns of NGAL induction and neutrophil accumulation are divergent. NGAL mRNA and protein expression was maximally noted at 12 hours of reflow, whereas neutrophil accumulation peaks at 24 hours by which time NGAL expression has significantly declined. Third, no NGAL-expressing neutrophils were detectable by immunofluorescence in the kidney samples examined). Fourth, NGAL mRNA and protein induction was documented to occur in cultured human proximal tubule cells following in vitro ischemia, with NGAL secreted into the culture medium within 1 hour of ATP depletion, in a system where neutrophils are absolutely absent. Nevertheless, some contribution from infiltrating neutrophils to the observed NGAL upregulation may have occurred. It is possible that upregulation of NGAL in renal tubule cells may be induced by local release of cytokines from neutrophils trapped in the microcirculation early after ischemic injury.

An older name for NGAL is HNL. Prior art patent 6,136,526 teaches a method for detecting HNL to distinguish a bacterial infection from a viral infection. Infections cause inflammation in the classical sense of induction of the immune system by attracting neutrophils and other immune cells to the site of infection. When the immune cells infiltrate the affected region, histamines and an array of proinflammatory cytokines are released in the intracellular spaces to induce phagocytosis and killing of the organisms. Activated neutrophils also secrete NGAL in response to bacterial but not viral infections. This differential response is likely to be due to a lipopolysaccharide (LPS) moiety on the surface of bacteria, since NGAL avidly binds LPS. NGAL will then diffuse into capillaries located close to an infected site and, when it reaches a sufficient level, can be detected in serum or plasma. It is not clear how soon neutrophils begin to secrete NGAL in response to bacterial infection, or how long it takes before NGAL released from neutrophils reaches detectable levels in serum.

This is in contrast to the present invention in several respects. First, there is little or no involvement of neutrophils or other immune cells in early ischemic or nephrotoxic injury. Second, ischemic or nephrotoxic injuries induce early and rapid expression of NGAL in cells of the affected tissues, such as those lining the various nephron segments. Third, the injured cells of the kidney release NGAL directly into the urine, where it appears within minutes of the injury. Fourth, the inflammation that typically occurs 6-12 hours after ischemic or nephrotoxic injury is distinct from that caused by an infection. Cell death induced by ischemic or nephrotoxic injury induces infiltrates primarily comprising macrophages that secrete proinflammatory cytokines to promote phagocytosis of cellular debris in the damaged tissue. Fifth, although some neutrophil accumulation has been shown to occur in animal models of ischemic kidney injury, this starts to occur only about 4 hours after the injury and peaks at about 24 hours after the injury. In contrast, urine NGAL peaks at 2-4 hours after the injury and is significantly diminished by 24 hours (1-3). Thus, the different time courses of urinary NGAL excretion and neutrophil accumulation argue against an inflammatory source for the urine and serum NGAL following ischemic injury. Sixth, although neutrophil accumulation has been shown in animal models, this has never been shown or substantiated in human acute renal failure. Seventh, we have documented NGAL accumulation in cultured kidney tubule cells following ischemic injury in vitro, in a system where neutrophils are absolutely absent. See Rabb H and Star R. Acute Renal Failure, Molitoris BA and Finn WF (editors), WB Saunders, Philadelphia, 2001, pp89-100; Chiao et al. J Clin Invest 1997; 99:1165-1172; and Rabb H et al. Am J Physiol 1996; 271 F408-F413.

An adequate explanation for the induction of NGAL by stimulated epithelia has been lacking, and whether NGAL is protective or proximate to injury or even an innocent bystander remains unclear. Recent evidence suggests that, at least in a subset of cell types, NGAL may represent a pro-apoptotic molecule. In the mouse pro-B lymphocytic cell line, cytokine withdrawal resulted in a marked induction of NGAL as well as onset of apoptosis. Purified NGAL produced the same pro-apoptotic response as cytokine deprivation, including activation of Bax, suggesting that NGAL is proximate to programmed cell death. NGAL has also been linked to apoptosis in reproductive tissues. Epithelial cells of the involuting mammary gland and uterus express high levels of NGAL, temporally coinciding with a period of maximal apoptosis. It is likely that NGAL regulates a subset of cell populations by inducing apoptosis. Stimulated epithelia may upregulate NGAL in order to induce apoptosis of infiltrating neutrophils, thereby allowing the resident cells to survive the ravages of the inflammatory response. Alternatively, epithelial cells may utilize this mechanism to regulate their own demise. However, it is interesting to note that induction of NGAL following renal ischemia-reperfusion injury occurs predominantly in the proximal tubule cells, and apoptosis under the same circumstances is primarily a distal tubule cell phenomenon.

Other recent studies have revealed that NGAL enhances the epithelial phenotype. NGAL is expressed by the penetrating rat ureteric bud, and triggers nephrogenesis by stimulating the conversion of mesenchymal cells into kidney epithelia. Another lipocalin, glycodelin, has been shown to induce an epithelial phenotype when expressed in human breast carcinoma cells. These findings are especially pertinent to the mature kidney, in which one of the well-documented responses to ischemic injury is the remarkable appearance of dedifferentiated epithelial cells lining the proximal tubules. An important aspect of renal regeneration and repair after ischemic injury involves the reacquisition of the epithelial phenotype, a process that recapitulates several aspects of normal development. This suggests that NGAL may be expressed by the damaged tubule in order to induce re-epithelialization. Support for this notion derives from the recent identification of NGAL as an iron transporting protein that is complementary to transferrin during nephrogenesis. It is well known that the delivery of iron into cells is crucial for cell growth and development, and this is presumably critical to postischemic renal regeneration just as it is during ontogeny. Since NGAL appears to bind and transport iron, it is also likely that NGAL may serve as a sink for iron that is shed from damaged proximal tubule epithelial cells. Because it has been observed that NGAL can be endocytosed by the proximal tubule, the protein could potentially recycle iron into viable cells. This might stimulate growth and development, as well as remove iron, a reactive molecule, from the site of tissue injury, thereby limiting iron-mediated cytotoxicity.

NGAL is a novel-serum biomarker for cisplatin-induced nephrotoxic renal injury that is more sensitive than previously described biomarkers. One example is kidney injury molecule-1 or KIM-1, a putative adhesion molecule involved in renal regeneration. In a rat model of cisplatin nephrotoxicity, KIM-1 was qualitatively detectable 24-48 hours after the initial insult, rendering it a somewhat late marker of tubular cell damage. NGAL is believed to be readily and quantitatively detected within 3 hours following cisplatin administration at doses known to result in renal failure. In addition, urinary and serum NGAL detection precede the appearance of other markers in the urine such as NAG. Appearance of NGAL in the urine and serum also precede the increase in serum creatinine that is widely used to diagnose nephrotoxic renal failure.

It is believed that serum NGAL is evident even after mild "sub-clinical" doses of cisplatin, in spite of normal serum creatinine levels. Thus, the invention has important implications for the clinical management of patients on cisplatin therapy. The efficacy of cisplatin is dose dependent, but the occurrence of nephrotoxicity frequently hinders the use of higher doses to maximize its antineoplastic potential. Nephrotoxicity following cisplatin treatment is common and may manifest after a single dose with acute renal failure. Although several therapeutic maneuvers have proven to be efficacious in the treatment of cisplatin-induced nephrotoxicity in animals, successful human experiences have remained largely anecdotal. One reason for this may be the lack of early markers for nephrotoxic acute renal failure, and hence a delay in initiating therapy. In current clinical practice, acute renal injury is typically diagnosed by measuring serum creatinine. However, it is well known that creatinine is an unreliable and delayed indicator during acute changes in kidney function. First, serum creatinine concentrations may not change until about 50% of kidney function has already been lost. Second, serum creatinine does not accurately depict kidney function until a steady state has been reached, which may require several days. Thus, the use of serum creatinine measurements impairs the ability to both detect and quantify renal damage during the early phases of renal injury. However, animal studies have suggested that while nephrotoxic acute renal failure can be prevented and/or treated, there is a narrow "window of opportunity" to accomplish this, and treatment must be instituted very early after the initiating insult. The lack of early biomarkers of renal injury has impaired the ability of clinicians to initiate potentially effective therapies in a timely manner. The use of NGAL in an assay system would also be of value for testing existing or emerging therapeutic or preventive interventions, and for the early evaluation of the nephrotoxic potential of other pharmaceutical agents. NGAL detection is a novel, non-invasive, early serum biomarker for cisplatin-induced kidney damage. Early detection may enable clinicians to administer timely therapeutic interventions, and to institute maneuvers that prevent progression to overt nephrotoxic renal failure.

The upregulation and serum transport of NGAL may represent a rapid response of renal tubule cells to a variety of insults, and the detection of NGAL in the serum may represent a widely applicable noninvasive clinical tool for the early diagnosis of tubule cell injury.

NGAL is a sensitive, noninvasive serum biomarker for renal tubular cell injuries, including renal ischemia and nephrotoxemia. The examination of the expression of NGAL in the serum of patients with acute, mild and early forms of renal tubular cell injury, using the rapid and simple detection methods and kits of the invention, can alert and enable clinicians to institute timely interventional efforts in patients experiencing acute renal failure, and to alert clinicians to institute maneuvers aimed at preventing progression in patients with subtle, subclinical renal tubular cell injuries (such as a nephrotoxins, kidney transplants, vascular surgery, and cardiovascular events) to overt ARF.

In the United States alone, there are approximately 16,000 kidney transplants performed every year. This number has been steadily increasing every year. About 10,000 of these are cadaveric kidney transplants, and are at risk for ARF. Each of these patients would benefit enormously from serial NGAL measurements, which could represent routine care.

Ischemic renal injury has also been associated with open heart surgery, due to the brief interruption in blood flow that is inherent in this procedure. The number of open heart surgeries performed annually can be estimated. In any moderately busy adult hospital, approximately 500 such operations are performed every year. Given that there are at least 400 such moderately busy hospitals in the United States alone, one can conservatively estimate that 200,000 open heart surgeries are performed every year. Again, serial NGAL measurements would be invaluable in these patients, and would represent the standard of care.

### EXAMPLES OF THE INVENTION

In the following examples of the invention, 71 children undergoing CPB were studied. Serial urine and blood samples were analyzed by Western blots and ELISA for NGAL expression. The primary outcome variable was acute renal injury, defined as a 50% increase in serum creatinine from baseline. Twenty patients (28%) developed acute renal injury, but the diagnosis using serum creatinine was possible only 1-3 days after CPB. In contrast, urine NGAL rose from a baseline of 1.6±0.3 ng/ml to 147±23 ng/ml at 2 hours after CPB. Serum NGAL increased from a baseline of 3.2±0.5 ng/ml to 61±10 ng/ml at 2 hours after CPB. Univariate analysis showed a significant correlation between acute renal injury and the following: 2 hour urine NGAL, 2 hour serum NGAL, and CPB time. By multivariate analysis, the urine NGAL at 2 hours post CPB emerged as the most powerful independent predictor of acute renal injury. A ROC curve for the 2-hour urine NGAL revealed an area under the curve of 0.998, and a sensitivity of 1.00 and specificity of 0.98 for a cutoff value of 50 ng/ml. Urine and serum NGAL were novel, sensitive, specific, highly predictive early biomarkers for acute renal injury following cardiac surgery.

*Study Design:* The investigation was approved by the Institutional Review Board of the Cincinnati Children's Hospital Medical Center. Written informed consent was obtained from the legal guardian of each patient before enrollment. All patients undergoing cardiopulmonary bypass (CPB) for surgical correction of congenital heart disease between January and November of 2004 were prospectively enrolled. Exclusion criteria included pre-existing renal insufficiency, diabetes mellitus, peripheral vascular disease, and the use of nephrotoxic agents before or during the study period. We therefore studied a homogeneous population of patients with very likely no major confounding variables in whom the only obvious renal insult would be the result of ischemia-reperfusion injury following CPB. To minimize post-operative volume depletion, all patients received at least 80% of their maintenance fluid requirements during the first 24 hours after surgery, and 100% maintenance subsequently. Spot urine and blood samples were collected at baseline and at frequent intervals for five days following CPB. Urine samples were obtained every two hours for the first 12 hours, and then once every 12 hours. Blood samples were collected at 2 hours post CPB, every 12 hours for the first day, and then once daily for five days. When the CPB time exceeded 2 hours, the first post-operative urine and serum samples were obtained at the end of CPB, and this sample was considered as the 2 hour collection. Urine and blood were also obtained from healthy adult volunteers for establishment of normal NGAL values. Samples were centrifuged at 2,000 g for 5 min, and the supernatants stored in aliquots at - 80°C. Serum creatinine was measured at baseline, and routinely monitored in these critically ill children at least twice a day in the immediate post-operative period, and at least daily after post-operative day three.

*Statistical Methods:* All results are expressed as means ± SE. The SAS 8.2 statistical software was utilized for the analysis. A two-sample t-test or Mann-Whitney Rank Sum Test was used to compare continuous variables, and the Chi-square test or Fisher's exact test as indicated were used to compare categorical variables. A conventional receiver operating characteristic (ROC) curve was generated for urine NGAL at 2 and 4 hours post CPB and for serum NGAL at 2 hours post CPB. These were utilized to determine the sensitivities and specificities at different cutoff levels for urine and serum NGAL. The area under the curve was calculated to determine the quality of NGAL as a biomarker. An area of 0.5 is no better than expected by chance, whereas a value of 1.0 signifies a perfect biomarker. Univariate and multivariate stepwise multiple logistic regression analyses were performed to assess predictors of acute renal injury. Potential independent predictor variables included age, gender, race, CPB time, previous heart surgery, urine output, urine NGAL at 2 hours post CPB, and serum NGAL at 2 hours post CPB. A *p* value of <0.05 was considered significant.

*Patient Characteristics:* The guardians of 100 patients provided their informed written consents for their children's participation in this study. Twenty nine patients were excluded, all because of nephrotoxin use (ibuprofen, ACE inhibitors, gentamicin, vancomycin) before or soon after the surgery. Thus, 71 patients were included in the study, whose demographic characteristics, diagnoses, and outcome variables are shown in Table 1, below. All subjects started with normal kidney function and essentially undetectable levels of NGAL in the urine and serum, just like healthy controls. This study design allowed for the determination of the precise timing of NGAL appearance in the urine and serum following CPB. The results indicate that NGAL is not only a powerful immediate early biomarker for acute renal injury, preceding any increase in serum creatinine by 1-3 days, but is also a valid discriminatory marker over the entire duration of the study.

**TABLE 1 TABLE 1. Patient characteristics and clinical outcomes. *p<0.05 versus controls.**

| **Characteristic** | **Control** | **Acute Renal Injury** |
|---|---|---|
| | **N = 51** | **N = 20** |
| Age (years) | 4.0±0.7 | 2.1±1.2* |
| Gender (% males) | 62% | 65% |
| Race (% Caucasian) | 88% | 85% |
| Previous Heart Surgery | 29% | 25% |
| 25%CPB Time (minutes) | 105±8.6 | 179±13.6* |
| Change in serum creatinine (%) | 7.7 ± 1.8 | 99 ± 9.3* ± |
| **Diagnosis (n)** | | |
| Ventricular septal defect | 9 | 3 |
| Tetralogy of Fallot | 3 | 9 |
| Atrial Septal Defect | 7 | 0 |
| Coarctation of Aorta | 5 | 1 |
| Aortic Stenosis | 6 | 0 |
| Hypoplastic Left Heart | 2 | 3 |
| AV Canal | 3 | 2 |
| Pulmonic Stenosis | 3 | 1 |
| Transposition of the great arteries | 4 | 0 |
| Tricuspid atresia | 3 | 0 |
| Double-outlet right ventricle | 2 | 0 |
| Anomalous left coronary artery | 1 | 0 |
| Cor Triatriatum | 0 | 1 |
| LV Outflow Tract Obstruction | 1 | 0 |
| Mitral Regurgitation | 1 | 0 |
| Aortic Regurgitation | 1 | 0 |
| **p*<0.05 versus controls. | | |

A major strength of this study is the prospective recruitment of a homogeneous cohort of children subjected to renal ischemia-reperfusion injury during surgical correction of congenital cardiac disease. The patients in these examples were devoid of common co-morbid variables such as atherosclerotic disease, diabetes, and nephrotoxin use, all of which can confound and vitiate the identification of early biomarkers for ischemic acute renal injury.

*Clinical Outcomes:* The primary outcome, acute renal injury, defined as a 50% or greater increase in serum creatinine from baseline, occurred in 20 out of 71 patients within a three-day period, yielding an incidence rate of 28%. Out of these, 8 patients displayed an increase in serum creatinine in the 24-48 hours post CPB, but in the other 12 patients, the increase was further delayed to the 48-72 hour period post CPB. Thus, the diagnosis of acute renal injury using currently accepted clinical practices could be made only days after the inciting event.

Based on the primary outcome, subjects were classified as "control" or "acute renal injury". There were no differences between the two groups in gender, race, or urine output. Other variables that were collected included age, CPB time, previous heart surgery, urine output, and urine creatinine. Children who developed acute renal injury tended to be younger and with longer CPB time, as shown above in TABLE 1. Acute renal injury was more common in patients with an underlying diagnosis of hypoplastic left heart, Tetralogy of Fallot, and AV canal, and was less common or absent in patients with atrial septal defect, ventricular septal defect, or valvular heart disease. The primary outcome variable was the development of acute renal injury, defined as a 50% or greater increase in serum creatinine from baseline.

### EXAMPLE 1

*Western Analysis For NGAL Expression And Quantitation:* Equal aliquots (30 µl) of each urine sample were boiled for 10 min in denaturing buffer and subjected to standard Western Blot analysis with an affinity purified goat polyclonal antibody raised against human NGAL (F-19, Santa Cruz Biotechnology). Simultaneous blots were prepared under identical conditions of transfer and exposure with known quantities of recombinant human NGAL, as standards for quantitation of urine NGAL as previously described by Mishra et al. in Am J Nephrol 2004;24:307-315. The laboratory investigators were blinded to the sample sources and clinical outcomes until the end of the study.

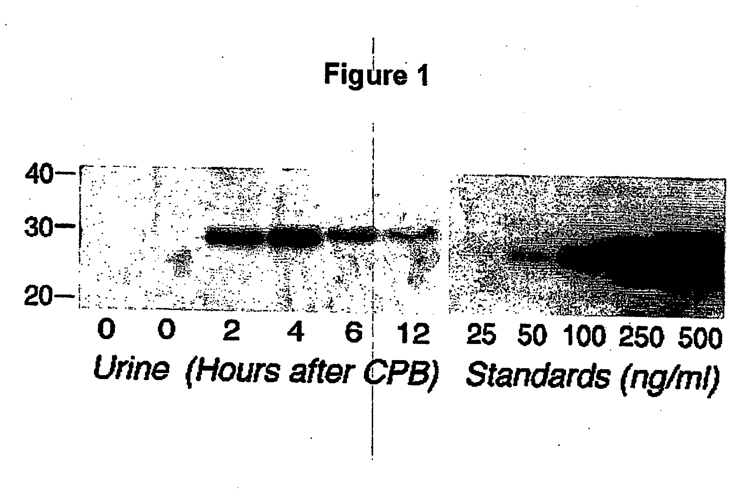

*Urine NGAL Measurements* - *Western Analysis:* NGAL was virtually undetectable in the urine of all patients prior to surgery, and in healthy volunteers (n=10). FIGURE 1 shows a Western Blot typical of that for a patient undergoing CPB. NGAL is not detected at 0 hours, or before CPB, but rapidly appears in the urine by 2 hours or less, and remains detectable by Western blot for at least 12 hours.

*ELISA For NGAL Quantitation:* A sensitive and reproducible ELISA for NGAL is an example of a method to provide accurate quantitation of the samples and to confirm the data obtained by Western analysis. Indeed, the ELISA results very closely paralleled those obtained by Western analysis, with a difference of less than 20%. The clinical utility of immunoblot-based techniques for the rapid detection of biomarkers for acute renal injury is limited by the time factor and variations in assay conditions. We modified previously published protocols for detection of NGAL derived from neutrophils as described by Kjeldsen et al. in J Immunol Methods 1996;198:155-164. Briefly, microtiter plates were coated overnight at 4°C with a mouse monoclonal antibody raised against human NGAL (#HYB211-05, Antibody Shop). All subsequent steps were performed at room temperature. Plates were blocked with buffer containing 1% BSA, coated with 100 µl of samples (urine or serum) or standards (NGAL concentrations ranging from 1-1000 ng/ml), and incubated with a biotinylated monoclonal antibody against human NGAL (#HYB211-01B, Antibody Shop) followed by avidin-conjugated HRP (Dako). TMB substrate (BD Biosciences) was added for color development, which was read after 30 min at 450 nm with a microplate reader (Benchmark Plus, BioRad). All measurements were made in triplicate, and in a blinded fashion.

### EXAMPLE 2

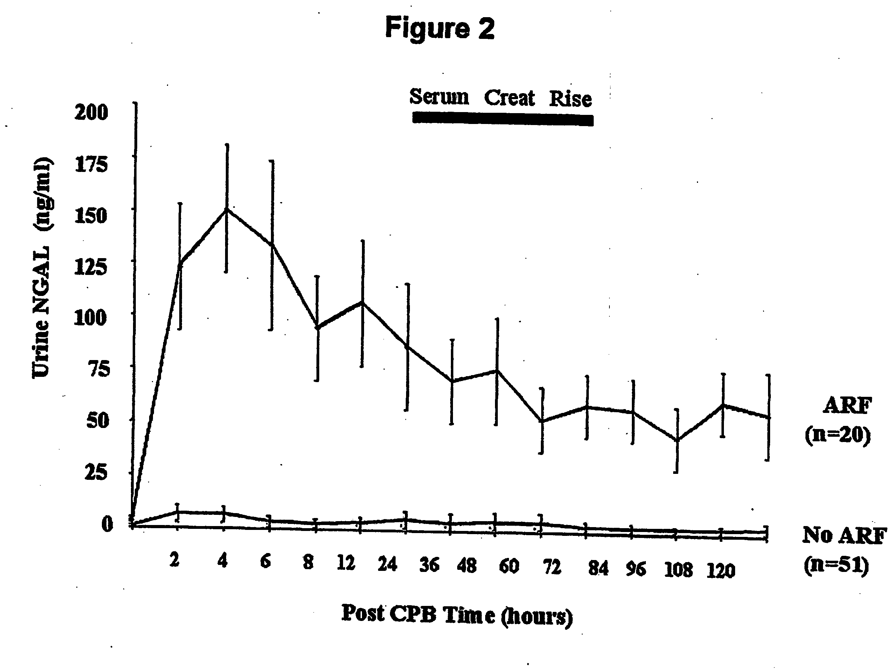

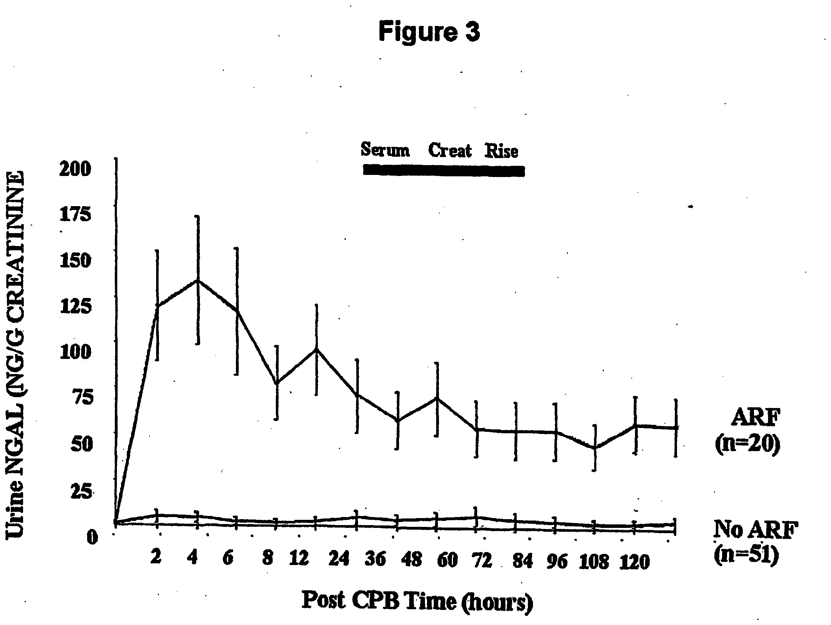

In patients who never developed acute renal injury, there was a small but statistically significant increase in urinary NGAL at 2 hours or the first available sample post CPB (4.9±1.5 ng/ml versus 0.9±0.3 ng/ml at baseline, *p*<0.05) and 4 hours post CPB (4.9±1.2 ng/ml, *p*<0.05 versus baseline). In marked contrast, patients who subsequently developed acute renal injury displayed a dramatic increase in urinary NGAL at all time points examined, as shown in FIGURE 2. The pattern of urinary NGAL excretion was characterized by a peak very early after the precipitating event (2-6 hours following CPB), followed by a lesser but sustained increase over the entire duration of the study. This overall pattern remained unchanged when urinary NGAL concentration was normalized for urinary creatinine excretion (FIGURE 3)..

### EXAMPLE 3

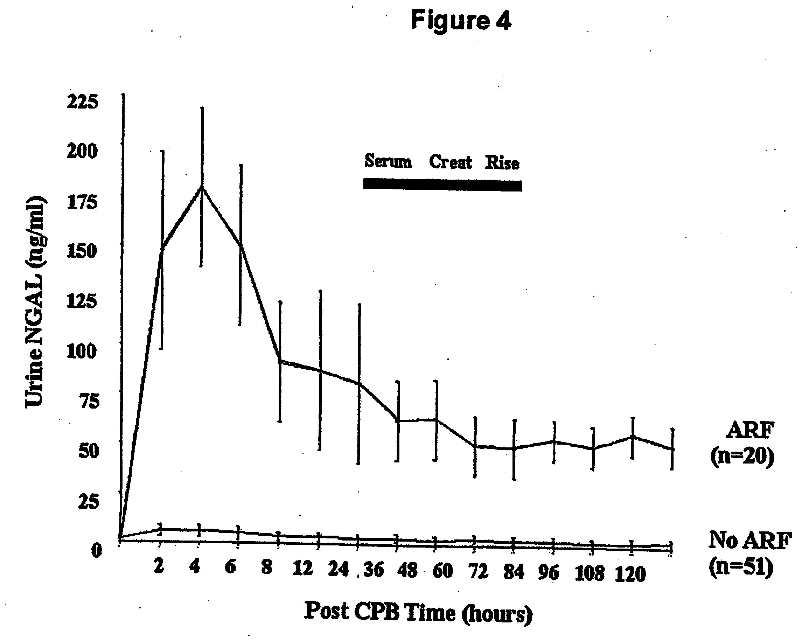

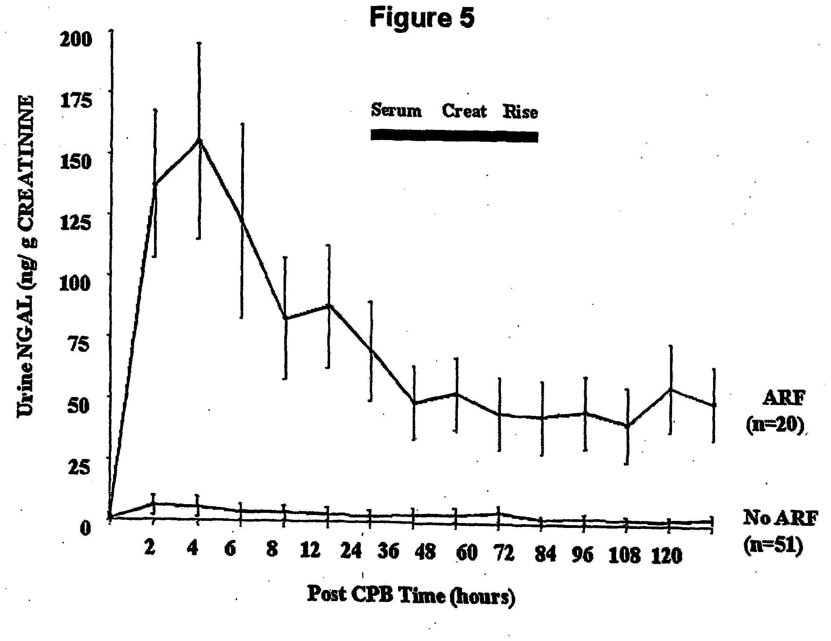

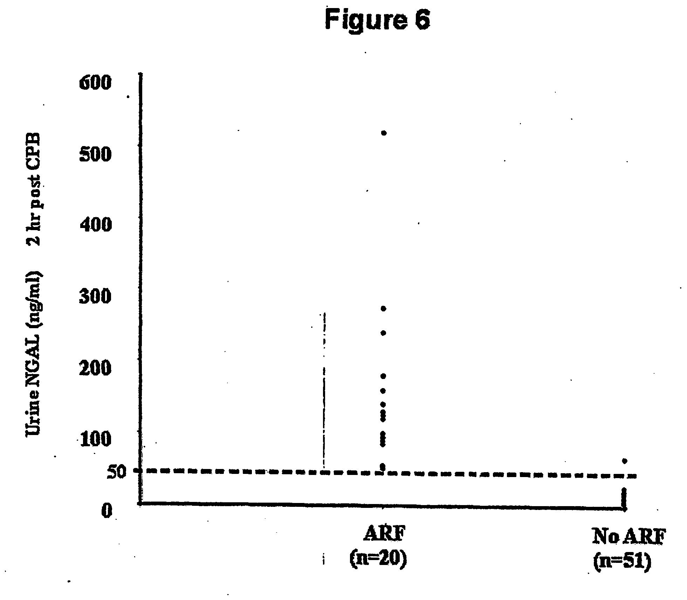

Urine NGAL levels were consistently low in healthy volunteers (2.2±0.5 ng/ml, n=10) and at baseline in all subjects (1.6±0.3 ng/ml, n=71). In patients who never developed acute renal injury, there was a small but statistically significant increase in urinary NGAL at 2 -hours-post CPB (5.9±1.4 ng/ml, *p*<0.05 versus baseline) and 4 hours post CPB (5.6±1.2 ng/ml, *p*<0.05 versus baseline). Patients who subsequently developed acute renal injury displayed a remarkable increase in urinary NGAL at all time points examined, as shown in FIGURE 4. Urinary NGAL excretion peaked very early after CPB, followed by a lesser but sustained increase over the entire duration of the study. Urinary NGAL levels were 147±23 ng/ml at 2 hours or the first available sample, 179±30 ng/ml at 4 hours, and 150±30 ng/ml at 6 hours post CPB in the acute renal injury group. This overall pattern remained consistent when urinary NGAL concentration was normalized for urinary creatinine excretion, i.e. 138±28 ng/mg creatinine at 2 hours, 155±40 ng/mg at 4 hours, and 123±35 ng/mg at 6 hours post CPB (FIGURE 5). A scatter plot of the first available post-operative urine NGAL measurements revealed that all 20 patients who subsequently developed acute renal injury displayed a level above an arbitrary cutoff value of 50 ng/ml, whereas only 1 out of 51 patients in the control group showed a urinary NGAL value above this arbitrary cutoff (FIGURE 6)

### EXAMPLE 4

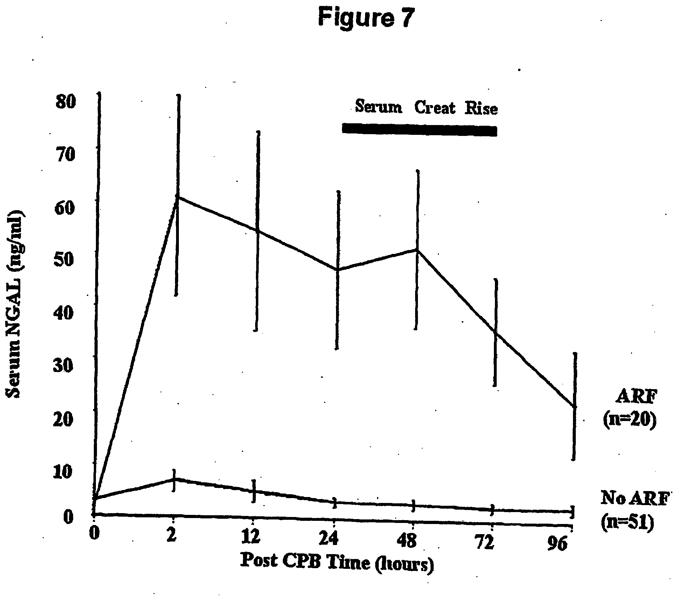

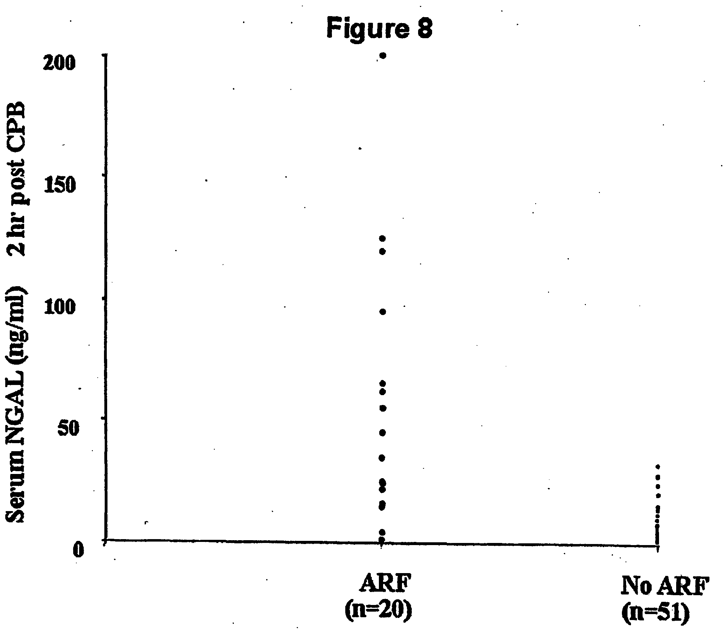

*Serum NGAL Measurements* - *ELISA:* Serum NGAL is a novel early biomarker of ischemic renal injury, similar to troponins in myocardial ischemia, and detection of serum NGAL is an example of the invention. Serum NGAL levels were consistently low in normal healthy volunteers (2.5t0.8, n=6) and all study subjects prior to surgery (3.2±0.5 ng/ml, n=71). Patients who never developed acute renal failure showed a small but statistically significant increase in serum NGAL at 2 hours or the first available sample post CPB (7.0±1.1 ng/ml, *p*<0.05 versus baseline) and 12 hours post CPB (5.2±0.8 ng/ml, *p*<0.05 versus baseline). Patients who subsequently developed acute renal failure displayed a striking increase in serum NGAL at all time points examined, as shown in FIGURE 7. Similar to urine NGAL, the serum NGAL peaked very early after CPB, followed by a lesser but sustained increase over the entire duration of the study. Serum NGAL levels were 61±10 ng/ml at 2 hours, 54.7±7.9 ng/ml at 12 hours, and 47.4±7.9 ng/ml at 24 hours post CPB in the acute renal failure group. A scatter plot of all the earliest serum NGAL measurements (2 hours post CPB) revealed that none of the 51 patients in the control group displayed a level above an arbitrary cutoff value of 50 ng/ml, whereas the majority of patients who developed acute renal failure showed a serum NGAL value above this value (FIGURE 8). The ELISA of the invention is an example of point-of care diagnostic kits for NGAL.

### EXAMPLE 5

*NGAL for Prediction of Acute Renal Injury:* A univariate analysis of the data revealed that the following outcomes were not predictive of acute renal failure: age, gender, race, previous surgery, and urine output. There was a significant correlation between acute renal failure (50% or greater in serum creatinine) and the following: urine NGAL at 2 hours or the first available sample post CPB (R=0.7.9, *p*<0.001), serum NGAL at 2 hours or the first available sample post CPB (R=0.64, *p*<0.001), and duration of CPB (R=0.49, *p*<0.001). However, by multiple stepwise regression analysis, only the urine NGAL at 2 hours post CPB emerged as the most powerful independent predictor of acute renal injury in this cohort (R=0.76, *p*<0.001).

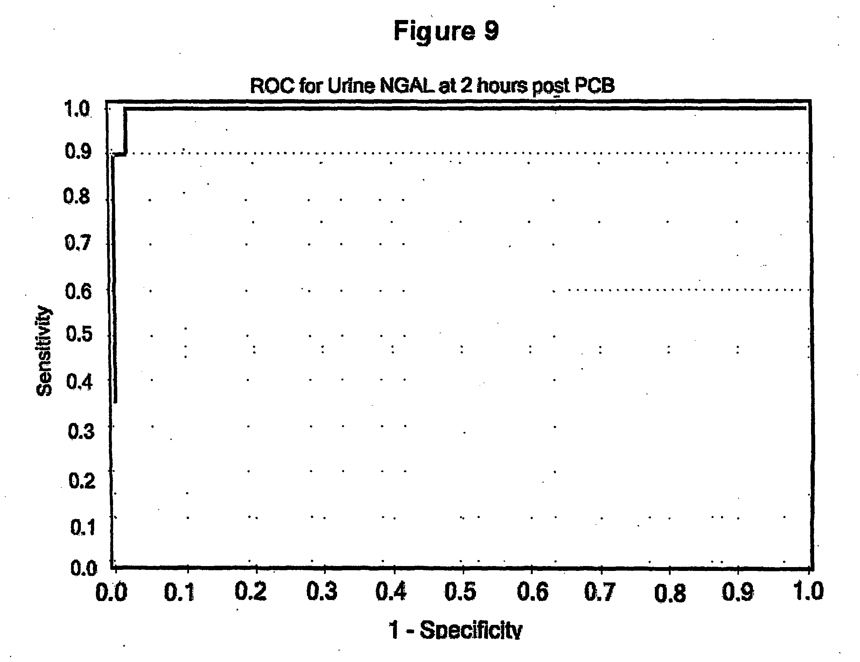

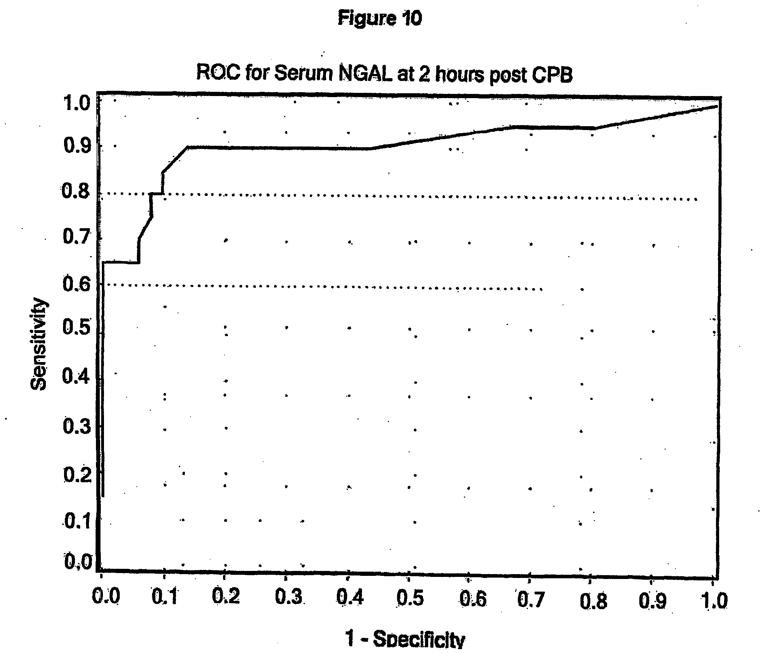

An ROC curve was constructed to determine the discriminatory power of urine and serum NGAL measurements for the early diagnosis of acute renal failure. For urine NGAL, the area under the curve was 0.998 at 2 hours post CPB (FIGURE 9), and 1.000 at 4 hours post CPB (not shown). For serum NGAL, the area under the curve was 0.906 at 2 hours post CPB (FIGURE 10). These values indicate that both urine and serum NGAL are excellent tests for the early diagnosis of acute renal failure. The derived sensitivities, specificities, and predictive values at different cutoff levels are listed in TABLE 2. For urine NGAL, a cutoff of either 25 or 50 ng/ml yields outstanding sensitivity and specificity at both 2 hours and 4 hours post CPB. For serum NGAL at 2 hours post CPB, sensitivity and specificity are optimal at the 25 ng/ml cutoff.

**TABLE 2. NGAL Test Characteristics at Various Cutoff Values.**

| Cutoffs for 2 hr Urine NGAL (ng/ml) | Sensitivity | Specificity | Positive Predictive Value | Negative Predictive Value |
|---|---|---|---|---|
| 25 | 1.00 | 0.98 | 0.95 | 1.00 |
| 50 | 1.00 | 0.98 | 0.95 | 1.00 |
| 80 | 0.90 | 1.00 | 1.00 | 0.96 |
| 100 | 0.70 | 1.00 | 1.00 | 0.89 |
| | | | | |

| Cutoffs for 4 hr Urine NGAL (ng/ml) | Sensitivity | Specificity | Positive Predictive Value | Negative Predictive Value |
|---|---|---|---|---|
| 25 | 1.00 | 0.96 | 0.91 | 1.00 |
| 50 | 0.95 | 1.00 | 0.95 | 0.98 |
| 80 | 0.70 | 1.00 | 1.00 | 0.89 |
| 100 | 0.65 | 1.00 | 1.00 | 0.88 |
| | | | | |

| Cutoffs for 2 hr Serum NGAL (ng/ml) | Sensitivity | Specificity | Positive Predictive Value | Negative Predictive Value |
|---|---|---|---|---|
| 25 | 0.70 | 0.94 | 0.82 | 0.89 |
| 50 | 0.50 | 1.00 | 1.00 | 0.84 |
| 80 | 0.20 | 1.00 | 1.00 | 0.76 |

NGAL is normally expressed at very low levels in several human tissues, including kidney, trachea, lungs, stomach, and colon (Cowland et al., Genomics 1997;45:17-23.). NGAL expression is markedly induced in injured epithelia. For example, NGAL concentrations are elevated in the serum of patients with acute bacterial infections, the sputum of subjects with asthma or chronic obstructive pulmonary disease, and the bronchial fluid from the emphysematous lung (Xu et al., Biochim Biophys Acta 2000;1482:298-307). The invention described herein stemmed from observations in animal models that NGAL is one of the earliest and most robustly induced genes and proteins in the kidney after ischemic, injury, and that NGAL is easily detected in the urine soon after ischemia. See Supavekin et al., Kidney Int 2003;63:1714-1724; Mishra et al., J Am Soc Nephrol 2003; 4:2534-2543; and Devarajan et al., Mol Genet Metab 2003;80:365-376. In the post-ischemic kidney, NGAL is markedly upregulated in several nephron segments and the protein accumulates predominantly in proximal tubules where it co-localizes with proliferating epithelial cells. These findings suggest that NGAL may be expressed by the damaged tubule in order to induce re-epithelialization. In support of this hypothesis is the recent identification of NGAL as a regulator of epithelial morphogenesis in cultured kidney tubule cells, and as an iron transporting protein during nephrogenesis (Gwira et. al., J Biol Chem 2005, and Yang et al., Mol Cell 2002; 10:1045-1056). It is well known that the delivery of iron into cells is crucial for cell growth and development, and this is presumably also critical to renal regeneration following ischemic injury. Indeed, recent findings indicate that exogenously administered NGAL ameliorates ischemic acute renal injury in mice by tilting the balance of tubule cell fate towards survival (Mishra et al., J Am Soc Nephrol 2004;15:3073-3082). Thus, NGAL has emerged as a center-stage player in the ARF field, not only as a novel biomarker but also as an innovative therapeutic maneuver.

While urinary diagnostics have several advantages, including the non-invasive nature of sample collection and the relatively few interfering proteins, some disadvantages also exist. These include the difficulty in obtaining urine samples from patients with severe oliguria, the potential changes in urinary biomarker concentration induced by the overall fluid status and diuretic therapy, and the fact that several urinary biomarkers have in the past shown insufficient sensitivity or specificity (Rabb H. Am J Kidney Dis 2003;42:599-600.). Serum-based diagnostics have revolutionized intensive care medicine. Recent examples include the measurement of troponins for the early diagnosis of and timely interventions in acute myocardial infarction and the prognostic value of B-type natriuretic peptide in patients with acute coronary syndrome (Hamm et al., N Eng J Med 1997;337:1648-1653; and De Lemos et al., N Engl J Med 2001;345:1014-1021). To our knowledge, NGAL is the only biomarker that has been examined in both serum and urine for the early diagnosis of ischemic renal injury.

The methods and use of the invention compare favorably with or surpass the usefulness of several other biomarkers for ischemic renal injury , such as those discussed in Hewitt et al., J Am Soc Nephrol 2004;15:1677-1689; Herget-Rosenthal et al., Kidney Int 2004;66:1115-1122; and Rabb, Am J Kidney Dis 2003;42:599-600). The majority of studies reported thus far have been retrospective, have examined biomarkers in the established phase of ARF, and have been restricted to only the urine and to only one method of detection. Several tubular proteins have been measured in the urine, with conflicting and unsatisfactory results (Westhuyzen et al., Nephrol Dial Transplant 2003;18:543-551; Herget-Rosenthal et al., Clin Chem 2004;50:552-558; Han et al., Kidney Int 2002;62:237-244). Kidney injury molecule-1 (KIM-1), a novel kidney-specific adhesion molecule, is detectable by ELISA in the urine of patients with established acute tubular necrosis. Also, the sodium hydrogen exchanger isoform 3 (NHE3) has been shown by Western blots to be increased in the membrane fractions of urine from subjects with established ARF (du Cheyron et al., Am J Kidney Dis 2003;42:497-506). However, the sensitivity and specificity of these biomarkers for the detection of renal injury have not been reported. Of the inflammatory cytokines involved in ARF, elevated levels of urinary IL-6, IL-8 and IL-18 have been demonstrated in patients with delayed graft function following cadaveric kidney transplants (35, 36). With the exception of NGAL, none of the biomarkers have been examined prospectively for appearance in the urine during the evolution of ischemic ARF. A recent prospective study has demonstrated that an increase in serum cystatin C precedes the increase in serum creatinine in a select patient population at high risk to develop ARF (Herget-Rosenthal et al., Kidney Int 2004;66:1115-1122). However, the ARF in these subjects was multifactorial, due to a combination of ischemic, prerenal, nephrotoxic, and septic etiologies. Furthermore, since cystatin C is primarily a marker of glomerular filtration rate (GFR), it can be inferred that serum cystatin C levels will rise only after the GFR begins to fall. On the other hand, NGAL is rapidly induced in the kidney tubule cells in response to ischemic injury, and its early appearance in the urine and serum is independent of the GFR, but is highly predictive of a fall in GFR that may occur several days later. A small transient increase in urine and serum NGAL in patients who did not develop ARF was consistent with previous observations that cardiopulmonary bypass surgery leads to release of NGAL into the circulation, probably secondary to inflammatory activation of leukocytes initiated by the extracorporeal circuit (Herget-Rosenthal et al., Kidney Int 2004;66: 1115-1122).

It is therefore intended that the protection herein be limited only by the appended claims.

## Claims

1. A method for the immediate or early-onset detection of a renal tubular cell injury (RTCI) in a mammal, the RTCI is acute and can be triggered by an ischemic renal injury or acute nephrotoxic injury, by determining the level of the immediate and early onset RTCI biomarker neutrophil gelatinase-associated lipocalin (NGAL) which appears within the first 24 hours of onset of RTCI in blood serum, comprising:
1) contacting a sample of blood serum obtained from a mammalian subject with an antibody for NGAL, to allow formation of a complex of the antibody and NGAL; and
2) detecting the antibody-NGAL complex.

2. The method of claim 1 further comprising:
3) determining that the mammal has the RTCI, based on the detected complex.

3. The method according to claim 1, wherein the NGAL appears in blood serum within the first 6 hours of, optionally within 2 hours of and preferably almost immediately after, the onset of RTCI.

4. The method according to claims 1 to 3, wherein the sample is obtained within 24 hours of the onset of the RTCI or within 24 hours after the suspected RTCI.

5. The method according to any one of claims 1 to 4, wherein the method is carried out on a plurality of samples from the subject obtained intermittently.

6. The method according to any one of claims 1 to 5, wherein the step of detecting the antibody-NGAL complex comprises contacting the complex with a second antibody for detecting the NGAL biomarker.

7. The method according to any one of claims 1 to 6, further comprising the step of detecting the level of NGAL in at least one sample obtained from the subject after the subject has received a treatment for the renal tubular cell injury.

8. The method according to any one of claims 1 to 7, wherein the step of detecting the antibody-NGAL complex comprises the steps of:
1) separating any unbound material of the sample from the capture antibody-NGAL complex;
2) contacting the capture antibody-NGAL complex with a second antibody for detecting NGAL, to allow formation of a complex between NGAL and the second antibody;
3) separating any unbound second antibody from the NGAL-second antibody complex; and
4) detecting the second antibody of the NGAL-second antibody complex.

9. The method of any one of claims 1 to 6, for identifying the extent of the renal tubular cell injury that has been caused by an event, wherein the extent of the renal tubular cell injury is identified based on the time for onset of the presence of NGAL in the sample, relative to the time of the event,
wherein the event preferably is selected from a surgical procedure, diminished blood supply to the kidneys, impaired heart function, surgical procedures, patients in intensive care units, and the administration of pharmaceuticals, radiocontrast dyes, or other medicament substances to the subject.

10. The method according to any one of claims 1 to 9, wherein the sample has a volume of up to 1 milliliter.

11. The method according to any one of claims 1 to 10, wherein the renal tubular cell injury is an acute ischemic injury.

12. The method according to any of claims 1 to 10, wherein the renal tubular cell injury is an acute nephrotoxic renal injury (NRI).

13. The method according to any one of claims 1 to 12, wherein the NGAL level is used to evaluate the renal tubular cell injury status of the subject.

14. The method according to any one of claims 1 to 10 for the rapid diagnosis of acute renal failure (ARF) in patients who are at risk of developing ARF in cardiac surgery.

15. The method according to any of claims 1 to 10, wherein the appearance of NGAL in serum precedes the increase in serum creatinine.

16. The method according to any of claims 1 to 10, for detecting the onset of RTCI caused by an event selected from the group consisting of all varieties of diminished blood supply to the kidneys, impaired heart function, surgical procedures, patients in intensive care units, and the administration of pharmaceuticals, radiocontrast dyes, or other medicament substances to a subject or nephrotoxins including cancer chemotherapy agents, antibiotics, antifungal agents, anti-inflammatory agents, immunosuppressants, and radiocontrast agents.

17. The method according to any one of claims 1 to 10, wherein the subject is a patient who is at risk of developing ARF.

18. The method according to any one of claims 1 to 10 for alerting a clinician to institute maneuvers aimed at preventing progression of a subtle, subclinical renal tubular cell injury to overt ARF.

19. The method according to any one of claims 1 to 10 for the early detection of the onset of renal tubular cell injury to reduce the time for treatment of the injury and reduce the risk of developing clinical acute renal failure (ARF).

20. The method according to any one of claims 1 to 10 for assessing the extent of renal injury wherein the extent of the renal injury is based on a proportional relationship between the extent of the injury with the quantity of NGAL present in the blood serum of the subject.

21. The method according to claim 20, wherein the extent of the renal injury ranges from the very onset of the RTCI to clinical acute renal failure (ARF).

## Patentansprüche

1. Verfahren zum Nachweisen einer Nieren-Tubuluszellen-Schädigung (renal tubular cell injury RTCI) bei einem Säugetier sofort oder früh nach dem Beginn, wobei die RTCI akut ist und durch eine ischämische Nierenschädigung oder eine akute nephrotoxische Schädigung ausgelöst werden kann, durch Bestimmen der Konzentration des sofortigen oder frühen Biomarkers für das Einsetzen einer RTCI, mit neutrophiler Gelatinase-assoziiertem Lipocalin (NGAL), das innerhalb der ersten 24 Stunden nach dem Einsetzen einer RTCI im Blutserum auftritt, aufweisend:
1) in Berührung Bringen einer Probe von Blutserum, die von einem Säugetier-Individuum erhalten wurde, mit einem Antikörper für NGAL, um die Bildung eines Komplexes des Antikörpers und NGAL zu erlauben; und
2) Nachweisen des Antikörper-NGAL-Komplexes.

2. Verfahren nach Anspruch 1 außerdem aufweisend:
3) Feststellen, dass das Säugetier die RTCI hat, auf der Basis des nachgewiesenen Komplexes.

3. Verfahren nach Anspruch 1, bei dem das NGAL innerhalb der ersten 6 Stunden, optional innerhalb von 2 Stunden, und bevorzugt nahezu sofort, nach dem Einsetzen einer RTCI im Blutserum auftritt.

4. Verfahren nach den Ansprüchen 1 bis 3, bei dem die Probe innerhalb von 24 Stunden nach dem Einsetzen der RTCI oder innerhalb von 24 Stunden nach der vermuteten RTCI erhalten wird.

5. Verfahren nach einem der Ansprüche 1 bis 4, bei dem das Verfahren an einer Mehrzahl von Proben durchgeführt wird, die periodisch von dem Individuum erhalten werden.

6. Verfahren nach einem der Ansprüche 1 bis 5, bei dem der Schritt des Nachweisens des Antikörper-NGAL-Komplexes ein in Berührung Bringen des Komplexes mit einem zweiten Antikörper zum Nachweisen des NGAL-Biomarkers umfasst.

7. Verfahren nach einem der Ansprüche 1 bis 6, außerdem aufweisend den Schritt des Nachweisens der Konzentration von NGAL in mindestens einer Probe, die von dem Individuum erhalten wurde, nachdem das Individuum eine Behandlung der Nieren-Tubuluszellen-Schädigung erhalten hat.

8. Verfahren nach einem der Ansprüche 1 bis 7, bei dem der Schritt des Nachweisens des Antikörper-NGAL-Komplexes folgende Schritte aufweist:
1) Abtrennen von jeglichem ungebundenem Material der Probe von dem Fangantikörper-NGAL-Komplex;
2) in Berührung Bringen des Fangantikörper-NGAL-Komplexes mit einem zweiten Antikörper zum Nachweisen von NGAL, um eine Bildung eines Komplexes zwischen NGAL und dem zweiten Antikörper zu erlauben;
3) Abtrennen von jeglichem ungebundenem zweiten Antikörper von dem NGAL-zweiter Antikörper-Komplex; und
4) Nachweisen des zweiten Antikörpers des NGAL-zweiter Antikörper-Komplexes.

9. Verfahren nach einem der Ansprüche 1 bis 6 zum Feststellen des Ausmaßes der Nieren-Tubuluszellen-Schädigung, die durch ein Ereignis verursacht wurde, wobei das Ausmaß der Nieren-Tubuluszellen-Schädigung festgestellt wird auf der Basis des Zeitpunkts des Einsetzens des Vorliegens von NGAL in der Probe relativ zum Zeitpunkt des Ereignisses,
wobei das Ereignis bevorzugt ausgewählt wird aus einem chirurgischen Eingriff, verringerter Blutzufuhr zu den Nieren, beeinträchtigter Herzfunktion, chirurgischen Eingriffen, Patienten in Intensivstationen und der Verabreichung von Pharmazeutika, Strahlenkontrast-Farbstoffen oder anderen Arzneimittelsubstanzen an das Individuum.

10. Verfahren nach einem der Ansprüche 1 bis 9, bei dem die Probe ein Volumen von bis zu 1 Milliliter hat.

11. Verfahren nach einem der Ansprüche 1 bis 10, bei dem die Nieren-Tubuluszellen-Schädigung eine akute ischämische Schädigung ist.

12. Verfahren nach einem der Ansprüche 1 bis 10, bei dem die Nieren-Tubuluszellen-Schädigung eine akute nephrotoxische Nierenschädigung (NRI) ist.

13. Verfahren nach einem der Ansprüche 1 bis 12, bei dem die NGAL-Konzentration verwendet wird, um den Nieren-Tubuluszellen-Schädigungszustand des Individuums zu bestimmen.

14. Verfahren nach einem der Ansprüche 1 bis 10 zur schnellen Diagnose von akutem Nierenversagen (acute renal failure ARF) bei Patienten, bei denen ein Risiko einer Entwicklung von ARF bei der Herzchirurgie besteht.

15. Verfahren nach einem der Ansprüche 1 bis 10, bei dem das Auftreten von NGAL im Serum dem Ansteigen von Serum-Creatinin vorausgeht.

16. Verfahren nach einem der Ansprüche 1 bis 10 zum Nachweisen des Einsetzens von RTCI, die von einem Ereignis verursacht wurde, das ausgewählt wird aus der Gruppe, die besteht aus allen Varianten von verringerter Blutzufuhr zu den Nieren, beeinträchtigter Herzfunktion, chirurgischen Eingriffen, Patienten in Intensivstationen und der Verabreichung von Pharmazeutika, Strahlenkontrast-Farbstoffen oder anderen Arzneimittelsubstanzen an das Individuum, oder von Nephrotoxinen einschließlich Krebs-Chemotherapiemitteln, Antibiotika, antimykotischen Mitteln, entzündungshemmenden Mitteln, Immunsuppressiva und Strahlenkontrastmitteln.

17. Verfahren nach einem der Ansprüche 1 bis 10, bei dem das Individuum ein Patient ist, bei dem das Risiko einer Entwicklung von ARF besteht.

18. Verfahren nach einem der Ansprüche 1 bis 10 zum Aufmerksam Machen eines Klinikers, Maßnahmen in die Wege zu leiten mit dem Ziel, ein Fortschreiten einer subtilen, subklinischen Nieren-Tubuluszellen-Schädigung zu offenem ARF zu verhindern.

19. Verfahren nach einem der Ansprüche 1 bis 10 zur Früherkennung des Einsetzens einer Nieren-Tubuluszellen-Schädigung, um die Zeit für die Behandlung der Schädigung zu verringern und das Risiko einer Entwicklung von klinischem akutem Nierenversagen (ARF) zu verringern.

20. Verfahren nach einem der Ansprüche 1 bis 10 zum Abschätzen des Ausmaßes einer Nierenschädigung, wobei das Ausmaß der Nierenschädigung auf einer proportionalen Beziehung zwischen dem Ausmaß der Schädigung und der Menge an NGAL, die im Blutserum des Individuums vorhanden ist, basiert.

21. Verfahren nach Anspruch 20, bei dem das Ausmaß der Nierenschädigung im Bereich vom allerersten Einsetzen der RTCI bis zu klinischem akutem Nierenversagen (ARF) liegt.

## Revendications

1. Procédé pour détecter de manière immédiate ou précoce une lésion des cellules tubulaires rénales (RTCI) chez un mammifère, la RTCI étant aiguë et pouvant être déclenchée par une lésion rénale ischémique ou une lésion néphrotoxique aiguë, par détermination du niveau de lipocaline associée à la gélatinase de neutrophiles (NGAL), biomarqueur de RTCI à apparition immédiate et précoce, qui apparaît dans le sérum sanguin dans les premières 24 heures suivant l'apparition d'une RTCI, comprenant :
1) la mise en contact d'un échantillon de sérum sanguin, obtenu à partir d'un sujet mammifère, avec un anticorps anti-NGAL, pour permettre la formation d'un complexe de l'anticorps et de la NGAL ; et
2) la détection du complexe anticorps-NGAL.

2. Procédé selon la revendication 1, comprenant en outre :
3) la détermination du fait que le mammifère a une RTCI, sur la base du complexe détecté.

3. Procédé selon la revendication 1, dans lequel la NGAL apparaît dans le sérum sanguin dans les 6 premières heures, éventuellement dans les 2 heures et de préférence presque immédiatement après l'apparition d'une RTCI.

4. Procédé selon les revendications 1 à 3, dans lequel l'échantillon est obtenu dans les 24 heures suivant l'apparition de la RTCI ou dans les 24 heures suivant une RTCI suspectée.

5. Procédé selon l'une quelconque des revendications 1 à 4, dans lequel le procédé est mis en oeuvre sur plusieurs échantillons provenant du sujet, obtenus de façon intermittente.

6. Procédé selon l'une quelconque des revendications 1 à 5, dans lequel l'étape de détection du complexe anticorps-NGAL comprend la mise en contact du complexe avec un deuxième anticorps pour détecter le biomarqueur NGAL.

7. Procédé selon l'une quelconque des revendications 1 à 6, comprenant en outre l'étape consistant à détecter le niveau de NGAL dans au moins un échantillon obtenu à partir du sujet après que le sujet a reçu un traitement contre la lésion des cellules tubulaires rénales.

8. Procédé selon l'une quelconque des revendications 1 à 7, dans lequel l'étape de détection du complexe anticorps-NGAL comprend les étapes consistant à :
1) séparer tout matériau non lié de l'échantillon d'avec le complexe anticorps de capture-NGAL ;
2) mettre en contact le complexe anticorps de capture-NGAL avec un deuxième anticorps pour détecter la NGAL, afin de permettre la formation d'un complexe entre la NGAL et le deuxième anticorps ;
3) séparer tout deuxième anticorps non lié d'avec le complexe NGAL-deuxième anticorps ; et
4) détecter le deuxième anticorps du complexe NGAL-deuxième anticorps.

9. Procédé selon l'une quelconque des revendications 1 à 6 pour identifier l'ampleur de la lésion des cellules tubulaires rénales qui a été provoquée par un événement, dans lequel l'ampleur de la lésion des cellules tubulaires rénales est identifiée sur la base du temps nécessaire à l'apparition de la présence de NGAL dans l'échantillon, par rapport au moment de l'événement, l'événement étant de préférence choisi parmi une opération chirurgicale, une diminution de l'apport de sang aux reins, une fonction cardiaque altérée, des opérations chirurgicales, des patients dans des unités de soins intensifs, et l'administration à un sujet de substances pharmaceutiques, produits de contraste, ou autres substances médicamenteuses.

10. Procédé selon l'une quelconque des revendications 1 à 9, dans lequel l'échantillon a un volume allant jusqu'à 1 millilitre.

11. Procédé selon l'une quelconque des revendications 1 à 10, dans lequel la lésion des cellules tubulaires rénales est une lésion ischémique aiguë.

12. Procédé selon l'une quelconque des revendications 1 à 10, dans lequel la lésion des cellules tubulaires rénales est une lésion rénale néphrotoxique (NRI) aiguë.

13. Procédé selon l'une quelconque des revendications 1 à 12, dans lequel le niveau de NGAL est utilisé pour évaluer l'état de la lésion des cellules tubulaires rénales chez le sujet.

14. Procédé selon l'une quelconque des revendications 1 à 10 pour le diagnostic rapide d'une insuffisance rénale aiguë (ARF) chez des patients présentant un risque de développer une ARF lors d'une chirurgie cardiaque.

15. Procédé selon l'une quelconque des revendications 1 à 10, dans lequel l'apparition de NGAL dans le sérum précède l'augmentation de la créatinine sérique.

16. Procédé selon l'une quelconque des revendications 1 à 10 pour détecter l'apparition d'une RTCI due à un événement choisi dans l'ensemble constitué par toutes les variétés d'apport sanguin réduit aux reins, de fonction cardiaque altérée, d'opérations chirurgicales, de patients dans des unités de soins intensifs, et d'administration à un sujet de substances pharmaceutiques, produits de contraste ou autres substances médicamenteuses, ou de néphrotoxines, y compris les agents chimiothérapeutiques anticancéreux, les antibiotiques, les agents antifongiques, les agents anti-inflammatoires, les immunodépresseurs, et les produits de contraste.

17. Procédé selon l'une quelconque des revendications 1 à 10, dans lequel le sujet est un patient présentant un risque de développer une ARF.

18. Procédé selon l'une quelconque des revendications 1 à 10 pour prévenir un clinicien qu'il faut entreprendre des manoeuvres visant à empêcher la progression d'une lésion des cellules tubulaires rénales subclinique subtile vers une ARF manifeste.

19. Procédé selon l'une quelconque des revendications 1 à 10 pour la détection précoce de l'apparition d'une lésion des cellules tubulaires rénales afin de réduire le temps de traitement de la lésion et réduire le risque de développement d'une insuffisance rénale aiguë (ARF) clinique.

20. Procédé selon l'une quelconque des revendications 1 à 10 pour déterminer l'ampleur d'une lésion rénale, dans lequel l'ampleur de la lésion rénale est basée sur une relation proportionnelle entre l'ampleur de la lésion et la quantité de NGAL présente dans le sérum sanguin du sujet.

21. Procédé selon la revendication 20, dans lequel l'ampleur de la lésion rénale est située dans la plage allant de l'apparition toute récente de la RTCI à une insuffisance rénale aiguë (ARF) clinique.
